# EUROPÄISCHE PATENTSCHRIFT

(11) **EP 1 483 264 B1**
(45) Veröffentlichungstag und Bekanntmachung des Hinweises auf die Patenterteilung: **27.12.2006**
(21) Anmeldenummer: 02798301.4
(22) Anmeldetag: 21.12.2002
(51) Int. Cl.: C07D 457/12, A61K 31/48, A61P 43/00, C07D 457/02, C07D 457/04, C07D 519/02

(54) **1-ALLYL-ERGOTALKALOID-DERIVATE- VERFAHREN ZU IHRER HERSTELLUNG UND IHRE VERWENDUNG ZUR PROPHYLAXE UND THERAPIE VON MIGRÄNE**
1-ALLYL ERGOT ALKALOID DERIVATIVES, METHOD FOR PRODUCING THE SAME AND THE USE THEREOF FOR PREVENTING AND TREATING MIGRAINE
DERIVES DE 1-ALLYL-ALCALOIDES ERGOT, LEUR PROCEDE DE PRODUCTION ET LEUR UTILISATION POUR PREVENIR ET SOULAGER DES MIGRAINES

(30) Priorität: 12.03.2002 DE 10212564
(43) Veröffentlichungstag der Anmeldung: 08.12.2004
(73) Patentinhaber: NeuroBiotec GmbH, 13353 Berlin (DE)
(72) Erfinder: FLIEGER, Miroslav, 1700 Praha 7 (CZ); PERTZ, Heinz, 10587 Berlin (DE); KRANDA, Karel, 14057 Berlin (DE); CVAK, Jan, 74601 Opava (CZ); GLUSA, Erika, D-99096 Erfurt (DE); JÄHNICHEN, Sven, 10587 Berlin (DE)
(74) Vertreter: Wablat, Wolfgang
(86) Internationale Anmeldenummer: PCT/DE2002/004759
(87) Internationale Veröffentlichungsnummer: WO 2003/076439

(56) Entgegenhaltungen:
- EP-A- 0 228 876
- CH-A- 392 537
- DE-A- 3 403 067
- DE-B- 1 076 137
- US-A- 3 232 942
- GIFFORD MARZONI ET AL.: "6-Methylergoline-8-carboxylic acid esters as serotonin antagonists: N1-substituent effectson 5-HT2 receptor affinity" JOURNAL OF MEDICINAL CHEMISTRY., Bd. 30, Nr. 10, - 1987 Seiten 1823-1826, XP002259098 AMERICAN CHEMICAL SOCIETY. WASHINGTON., US ISSN: 0022-2623
- CHEMICAL ABSTRACTS, vol. 136, Columbus, Ohio, US; abstract no. 325202n, BACHECHI, FIORELLA ET AL.: "Molecular complexes for the study of enantiodiscriminative processes" XP002259101 & STRUCTURAL CHEMISTRY, Bd. 13, Nr. 1, - 2002 Seiten 41-51, -& DATABASE CAPLUS [Online] CHEMICAL ABSTRACTS SERVICE, COLUMBUS, OHIO, US; Database accession no. 2002:102497 XP002259105
- CHEMICAL ABSTRACTS, vol. 128, Columbus, Ohio, US; abstract no. 154269t, BACHECHI, FIORELLA ET AL.: "Molecular structure of a chromatographic chiral selector based on a terguride derivative." XP002259102 & STRUCTURAL CHEMISTRY, Bd. 9, Nr. 1, - 1998 Seiten 39-45, -& DATABASE CAPLUS [Online] CHEMICAL ABSTRACTS SERVICE, COLUMBUS, OHIO, US; Database accession no. 1998:43663 XP002259106
- BENNO A. INGELSE ET AL.: "Reversed determination of the formation constants of 1-allyl terguride with mandelic acid optical isomers using capillary electrophoresis" JOURNAL OF CHROMATOGRAPHY A, Bd. 772, Nr. 1+2, - 1997 Seiten 179-184, XP002259099
- CHEMICAL ABSTRACTS, vol. 126, Columbus, Ohio, US; abstract no. 138017n, BACHECHI, FIORELLA ET AL.: "Complex of lisuride derivative and (S)-naproxen" XP002259103 & ACTA CRYSTALLOGRAPHICA, SECTION C: CRYSTAL STRUCTURES COMMUNICATIONS, Bd. c53, Nr. 1, - 1997 Seiten 136-140, -& DATABASE CAPLUS [Online] CHEMICAL ABSTRACTS SERVICE, COLUMBUS, OHIO, US; Database accession no. 1997:117950 XP002259107
- BENNO A. INGELSE ET AL.: "Ergot alkaloids as chiral selectors in capillary electrophoresis. Determination of the separation mechanism" JOURNAL OF CHROMATOGRAPHY A, Bd. 755, Nr. 2, - 1996 Seiten 251-259, XP002259100
- CHEMICAL ABSTRACTS, vol. 100, Columbus, Ohio, US; abstract no. 185414n, EICH, ECKART ET AL.: "New antibiotics with cytostatic activity" XP002259104 & BIOCHEM. PHARMACOL., Bd. 33, Nr. 4, - 1984 Seiten 523-526, -& DATABASE CAPLUS [Online] CHEMICAL ABSTRACTS SERVICE, COLUMBUS, OHIO, US; Database accession no. 1984:185414 XP002259108

## Beschreibung

Die vorliegende Erfindung betrifft eine neue Klasse von Ergotalkaloid-Derivaten mit selektiven antagonistischen Eigenschaften auf 5-HT₂-Rezeptoren zur Prophylaxe und Linderung von migränebedingtem Kopfschmerz, M. Parkinson, Störungen der Thrombozytenfunktion u.a.

### Hintergrund und Stand der Technik

Migräne ist eine der am häufigsten vorkommenden Erkrankungen. An ihr leiden ca. 10% der Bevölkerung (Worthington, 1996, Current migraine theory, Can. J. Clin. Pharm., 3, 39-51). Damit gehört sie zu den großen Volkskrankheiten.

Charakteristisches Merkmal einer klassischen Migräne ist das Auftreten einer Frühphase, die zunächst mit Sehstörungen, der sog. visuellen Aura, einhergeht (K. Kranda, J.J. Kulikowski, 1984, Visual Aura in classical migraine, in: Neurobiology of Pain, Eds. Holden & Wilmslow, MUP) und für Minuten und manchmal Stunden anhalten kann. Der visuellen Aura können u.U. ein- oder zweiseitige, pulsierende Schmerzen folgen. Das Auftreten der visuellen Aura, das von nur etwa 20% der Migräneleidenden berichtet wird, definiert die sogenannte "klassische Migräne" (M.L. Leone et al., 1995, A review of the treatment of primary headaches, Ital. J. Neurol. Sci., 16, 577-586). Auf die visuelle Aura müssen nicht in jedem Fall Kopfschmerzen folgen, ebenso wie manche Patienten bei verschiedenen Vorfällen Schmerzattacken mit oder ohne Aura erfahren (W.F. Stewart et al., 1992, Prevalence of migraine headache in the United States, J.A.M.A., 267, 64-69); J. Olesen et al., 1994, Migraine classification and diagnosis, Neurol., 44, 56-510).

Zusammenfassend kann gesagt werden, dass die klassische Migräne aus zwei Hauptphasen besteht: a) der nicht immer auftretenden auralen Phase und b) der akuten schmerzhaften, durch Kopfschmerz geprägten Phase. Letztere Kopfschmerzphase ist durch pulsartigen Schmerz und Übelkeit charakterisiert. Sie wird öfters von Licht- oder Lärmscheu begleitet und kann bis zu Tagen andauern (P.J. Goadsby, 1997, Current concepts of the pathophysiology of migraine, Neurol. Clin., 15, 27-42).

Die Ursachen und Mechanismen der Migräne sind bislang nicht vollständig geklärt. Eine sich ausbreitende Depression in der Hirnrinde wurde als Initiator der visuellen Aura der klassischen Migräne vorgeschlagen (Lauritzen, 1994, Brain, 117, 199-210). Jedoch lassen theoretische Betrachtungen der Cytoarchitektur des Sehzentrums eine solche Hypothese unplausibel erscheinen (K. Kranda, J.J. Kulikowski, 1984, Visual Aura in classical migraine, in: Neurobiology of Pain, Eds. Holden & Wilmslow, MUP).

Bislang existiert eine Vielzahl vorgeschlagener oder bereits zur Behandlung und Vorbeugung eingesetzter Medikamente. Genannt seien Analgetika, Antihistamine, Calcium-Kanalblocker und die Gruppe der Serotonin-Agonisten/ Antagonisten, wie die Ergot-Alkaloide, Sumatriptan, Pizotifen und Propanolol. Weitere pharmakologische Klassen sind potentiell zur Migränebehandlung und Prophylaxe verwendbar, so z.B.

Gefäßerweiterer, Neuroleptika, β-Rezeptoren-Blocker und auch Antiepileptika, wie Natriumvalproat.

Obwohl in den letzten Jahren beachtliche Fortschritte in der Migränebehandlung erzielt wurden, wie z.B. mit dem 5-HT₁-Agonisten Sumatriptan, wird dieses Leiden oft noch falsch diagnostiziert und unangemessen behandelt (Worthington, 1996, Can. J. Clin. Pharm., 3, 39-51). Keine der bislang existierenden Behandlungen bewirkt eine dauerhafte Linderung des Migräne-Kopfschmerzes. Zum Beispiel tritt nach der Verabreichung von Sumatriptan die Krankheit in 40% aller Fälle innerhalb eines Zeitraumes von 24 Stunden wieder auf. Alle bisher therapeutisch verwendeten Substanzen sind in Beziehung auf Serotonin-Rezeptoren sehr wenig spezifisch, und dies führt zu Nebenwirkungen (z.B. Koronarkonstriktion der Gefäße).

5-HT_{2B}-Antagonisten wird ein großes Potential für die Behandlung und Prophylaxe von Migräne zugeschrieben. Es wurde beobachtet, dass 5- HT_{2B}-Agonisten, wie z.B. m-Chlorphenylpiperazin, Migräneattacken in empfindlichen Individuen hervorrufen können (Fozard and Gray, 1989, Trends Pharmacol. Sci, 10(8), 307-309). Umgekehrt sind HT_{2B}-Antagonisten in der Lage, das Ausbrechen einer Migräne zu verhindern (Kalkman, 1994, Life Sci., 54, 641-644).

Die bisher effektivsten Arzneimittel in der Migräneprophylaxe, Methysergid, Pizotifen und Propanolol, haben einen antagonistischen Effekt auf 5-HT_{2B}-Rezeptoren (Kalkman, 1994, Life Sci., 54(10), 641-644). Auch Methylergometrin als hauptsächlich aktiver Metabolit von Methysergid im Menschen besitzt eine starke antagonistische Wirkung auf 5-HT_{2B}-Rezeptoren (Fozard and Kalkman, 1994, Arch. Pharmakol. 350(3), 225-229). 5-HT_{2B}-Rezeptoren wurden in Endothelzellen (Gefäßinnenhaut) lokalisiert, so auch in den Endothelzellen der Blutgefäße des Gehirns (Ullmer et. al, 1995, FEBS-Lett., 370(3), 215-221), welche die Gefäßrelaxation durch Freisetzung von Stickoxid (NO) vermitteln. NO spielt möglicherweise als Initiator der Migräne eine Rolle (Olesen et al., 1994, Ann. Neurol., 28, 791-798). Alle genannten Migränemittel weisen Nachteile auf oder zeigen erhebliche Nebenwirkungen.

Gleichfalls erwünscht für eine Anti-Migränewirkung ist die hohe Wirksamkeit der beschriebenen Substanzen auf Thrombozyten, da bei Migräne verschiedene Störungen der Thrombozytenfunktion bekannt sind. Diese äußern sich in vermehrter Adhäsion und Aggregation bei einem Anfall, einem "Sludging" und hieraus resultierender gestörter Mikrozirkulation, sowie einer Freisetzung von Serotonin, Thromboxan A und anderer vasoaktiver Substanzen (Übersicht bei S. Diamond, Migraine Prevention and Management, Marcel Dekker, Basel, 1990). Die Wirkungen auf Thrombozyten sind auch unabhängig von der Migräne in allen Situationen gestörter oder gesteigerter Thrombozytenaggragation erwünscht.

Der vorliegenden Erfindung liegt somit die Aufgabe zugrunde, ein Arzneimittel zur Linderung und Prophylaxe von Migräneerkrankungen bereitzustellen.

Erfindungsgemäß werden neue Allyl-Ergotalkaloid-Derivate der allgemeinen Formel (I) zur Verfügung gestellt 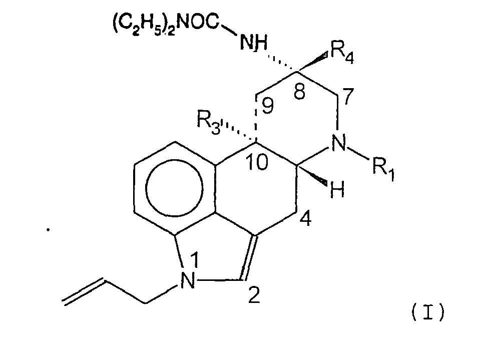 in welcher
R₁ für eine Ethyl-, n-Propyl-, i-Propyl- oder Allylgruppe,
R₃ für Wasserstoff oder eine -OCH₃ -Gruppe, und
R₄ für Wasserstoff, eine Methyl-, Ethyl-, n-Propyl-, i-Propyl- oder -CH₂OH-Gruppe, steht, und
die Bindung zwischen den C-Atomen 9 und 10 entweder eine Einfachbindung oder eine Doppelbindung bei gleichzeitigem Wegfall des Restes R₃ ist.

Die erfindungsgemäßen Verbindungen der allgemeinen Formel (I) werden hergestellt, indem eine Verbindung der allgemeinen Formel (III) 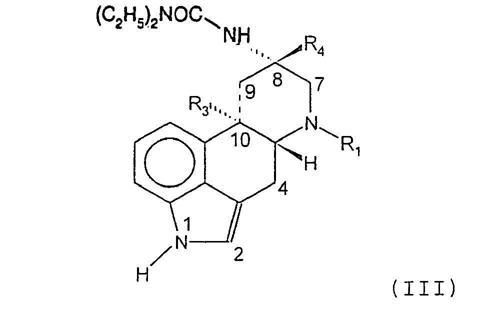 in welcher
R₁ für eine Ethyl-, n-Propyl-, i-Propyl- oder Allylgruppe,
R₃ für Wasserstoff oder eine -OCH₃ -Gruppe, und
R₄ für Wasserstoff, eine Methyl-, Ethyl-, n-Propyl-, i-Propyl- oder -CH₂OH-Gruppe, steht, und
die Bindung zwischen den C-Atomen 9 und 10 entweder eine Einfachbindung oder eine Doppelbindung bei gleichzeitigem Wegfall des Restes R₃ ist,
mit Allylbromid in CH₂Cl₂ gegebenenfalls unter Zugabe von Tetraethylammoniumhydroxid und NaOH umgesetzt wird.

Gegebenenfalls werden die erhaltenen erfindungsgemäßen Allyl-Ergotalkaloid-Derivate der allgemeinen Formel (I) nach üblichen Verfahren in ein Säureadditionssalz überführt, vorzugsweise in das Sulfat, Bisulfat, Nitrat, Phosphat, Hydrogenphosphat, Hydrochlorid, Hydrobromid, Hydrojodid, Acetat, Tartrat, Lactat, Citrat, Gluconat, Fumarat, Maleat, Hydroxylmaleat, Succinat, Pamoat, Benzoat, Propionat, Pyruvat, Oxalat, Malonat, Cinnamat, Salicylat, Alkylsulfonat, Arylsulfonat und Aralkylsulfonat.

Gegenstand der vorliegenden Erfindung sind außerdem Arzneimittel, die eih oder mehrere Allyl-Ergotalkaloid-Derivate der allgemeinen Formel (I) enthalten. Diese besitzen selektive antagonistische Eigenschaften auf 5-HT_{2A}- und 5-HT_{2B}-Rezeptoren.

Durch die an N¹ eingeführte Allylgruppe zeigen die erfindungsgemäßen Verbindungen eine überraschende Stabilität gegen metabolischen Abbau, wodurch die Wirkungsdauer als Arzneimittel verlängert wird. Ferner ist die Wirkungsselektivität auf 5-HT_{2B}- oder 5-HT_{2A}-Rezeptoren durch die Einführung der Allylgruppe höher als bei bisher bekannten Verbindungen. Im Falle von 1-Allylergotamin und 1-Allyldihydroergotamin zeigt sich die höhere Spezifität auch im Verlust der partialagonistischen Komponente der Ausgangssubstanzen.

Durch ihre selektiven antagonistischen Eigenschaften auf 5-HT_{2A}- und 5-HT_{2B}-Rezeptoren sind die erfindungsgemäßen Verbindungen als Arzneimittel insbesondere zur Behandlung und Prophylaxe von Migräne geeignet. Dabei ist die eine oder die andere Eigenschaft bei spezifischer Ausprägung besonders geeignet, im akuten Anfall zu wirken, wo es nach Diener bzw. H. Raskin und O. Appenzeller zur verstärkten Aktivierung des serotoninergen N. dorsalisraphe kommt, bzw. in der Anfallsprophylaxe, wo Serotonin-Rezeptoren an Gefäßen eine besondere Rolle spielen.

Ferner wirken die erfindungsgemäßen Substanzen hemmend auf die Aggregation von Thrombozyten. Neben der Behandlung der Migräne und begleitender Störungen der Thrombozytenfunktion sind die erfindungsgemäßen Substanzen auch allgemein und unabhängig von der Migräne zur Behandlung gestörter oder gesteigerter Thrombozytenaggragation geeignet.

Die erfindungsgemäßen Substanzen besitzen ferner eine sehr hohe Affinität zu verschiedenen Dopaminrezeptoren und vor allem zu dem D₂-Rezeptor. Dadurch sind sie auch für die Therapie von M.-Parkinson und anderer Dopaminmangelzustände, sowie des Restless-Legs-Syndroms und von Hyperprolaktinämie geeignet. Durch partialagonistische und -antagonistische Wirkungen sind die erfindungsgemäßen Substanzen zusätzlich zur Anwendung als Antipsychotika geeignet.

Ebenso sind die erfindungsgemäßen Allyl-Ergotalkaloid-Derivate der allgemeinen Formel (I) zur Prophylaxe und/oder zur Behandlung psychischer Erkrankungen und allgemeiner Erkrankungen des Nervensystems geeignet.

Gegenstand der vorliegenden Erfindung sind Arzneimittel zur oralen, sublingualen, transdermalen, rektalen, topicalen und parenteralen (z.B. intravenösen) Applikation, die neben üblichen Träger- und Hilfsstoffen eine Verbindung der allgemeinen Formel (I) oder deren pharmazeutisch verträgliches Säureadditionssalz als Wirkstoff enthalten.

Die Verabreichung des erfindungsgemäßen Arzneimittels kann in einer speziellen Depotform erfolgen, wie z.B. in Form einer kontrollierten Freisetzung des Wirkstoffs, einer anhaltenden Freisetzung, z.B. als transdermales Pflaster, einer stoßweisen Freisetzung, einer verzögerten Freisetzung oder einer doppelten Freisetzung.

Die Dosierung des erfindungsgemäßen Arzneimittels ist u.a. von dem zu behandelten Subjekt, der Stärke der Beschwerden und der Art der Verabreichung abhängig. Die effektive Dosis für die orale, sublinguale, transdermale, rektale, topicale und parenterale Verabreichung beträgt vorzugsweise 0,001-20 mg pro kg Körpergewicht und Tag. Die Arzneimittel der Erfindung werden unter Verwendung der üblichen festen oder flüssigen pharmazeutischtechnischen Zusatz- und Hilfsstoffe entsprechend der gewünschten Applikationsart auf bekannte Art und Weise hergestellt.

Als Zusatz- und Hilfsstoffe können beispielsweise Bindemittel, Füllstoffe, Tablettierhilfsmittel, Verdünnungsmittel, Lösungsvermittler, Farbstoffe, Geschmacksstoffe, Benetzungsmittel, Emulgatoren, pH-Pufferzusätze, Suspensionshilfsmittel, nichtwässrige Hilfsstoffe und Konservierungsmittel verwendet werden.

Ein Füllstoff kann beispielsweise aus Cellulose, Mannitol und Lactose ausgewählt sein. Als Lösungsvermittler können beispielsweise Stärke, Stärkederivate und Polyvinylpyrrolidon verwendet werden. Vorteilhaft ist die Zugabe von EDTA zu einer Lösung des Wirkstoffs. Ein Emulgator kann aus Natriumlaurylsulfat, Lecitin, Sorbitan-monooleat und Gummiarabicum ausgewählt sein. Ein Suspensionshilfsmittel kann z.B. aus Sorbitol, Methylcellulose, Gelatine, Hydroxyethylcellulose, Carboxymethylcellulose, Aluminiumstearatgel und den hydrierten Speisefetten ausgewählt sein. Als nichtwässrige Hilfsstoffe kommen unter anderen Mandelöl, Kokosnussöl, Glycerinester, Propylenglycol und Ethylalkohol in Betracht. Ein Konservierungsmittel kann beispielsweise aus Methyl-p-hydroxybenzoat, Ethyl-p-hydroxybenzoat, Bisulfit und Ascorbinsäure ausgewählt sein. Als Schmierstoff ist beispielsweise Magnesiumstearat verwendbar.

Eine orale Applikation kann beispielsweise in fester Form, als Tabletten, Kapseln, Granulationen, Pulver und Pastillen, oder in flüssiger Form, als wässrige Lösung, Suspension, Sirup oder lösbares Pulver erfolgen. Ebenso ist die Verabreichung in Form eines oralen Sprays möglich.

Eine parenterale Applikation kann in Form einer subcutanen, intramuskulären oder intravenösen Injektion erfolgen. Der Wirkstoff der allgemeinen Formel (I) in Form einer flüssigen Suspension oder in gelöster Form vorliegen, wobei er auch erst kurz vor der Injektion aufgelöst oder suspendiert werden kann. Der Zusatz von Emulgatoren oder Benetzern kann im Falle einer Suspension eine gleichmäßige Verteilung des Wirkstoffs in der Flüssigkeit bewirken. Als flüssiger Trägerstoff kommen u.a. Salzlösungen oder Glycerin in Betracht. Die Konzentration des Wirkstoffs beträgt vorzugsweise 0,1-10%.

Für eine transdermale Applikation wird der Wirkstoff der allgemeinen Formel (I) in einer Trägermatrix verteilt, die aus beispielsweise aus Mineralöl, Paraffinöl, einem Polyacrylat oder einem Wachs ausgewählt ist. Eventuell kann ein transdermaler Transportbeschleuniger und/oder Strukturbrecher zugesetzt werden, der z.B. aus Dimethylsulfoxid oder Propylenglycol ausgewählt ist.

### VERGLEICHSBEISPIELE

Die folgenden Beispiele dienen zur Verdeutlichung der vorliegenden Erfindung, ohne sie einzuschränken.

### Beispiel 1: Synthese und Charakterisierung der erfindungsgemäßen Verbindungen der Formel (I)

Eine Lösung von (5R,8S,10R)-Tergurid (4g,11.7 mM) in CH₂Cl₂ (160 ml) wurde mit einer 20%igen (v/v) Lösung von Tetraethylammoniumhydroxid (8ml) in 24 ml 50% (w/v) NaOH gemischt. Anschließend wurde tropfenweise Allylbromid (5ml, 58.6 mM) unter kräftigem Rühren zugegeben. Nach vollständiger Zugabe des Allylbromids wurde noch 5 Minuten weitergerührt. Nach Abtrennung wurde die organische Phase mit zweimal 200 ml Wasser gewaschen und unter Vakuum aufkonzentriert. Der Rückstand wurde auf einer Silica-gel-Säule (40 g) mit CH₂Cl₂ als Laufmittel chromatographiert. Die Fraktionen mit Allyltergurid wurden getrocknet und das Reinprodukt aus einer Diethylether/Petrolether-Lösung kristallisiert. Die Ausbeute betrug 2.5 g 1-Allyl-(5R,8S,10R)-tergurid.

Nach dem analogen Verfahren wurden alle weiteren Verbindungen der allgemeinen Formel (I) synthetisiert.

Die Strukturaufklärung wurde mit Massenspektroskopie und ¹H und ¹³C-NMR vorgenommen. Die Massenspektren wurden mit einem Gerät der Marke Finnigan MAT 90 (doppelfokussierend, BE Geometrie) unter folgenden Bedingungen aufgenommen:
lonisierungsenergie: 70 eV
Temperatur der Ionenquelle: 250°C
Emissionsstrom: 1 mA
Beschleunigungsspannung: 5 kV; DIP: 170°C

¹H und ¹³C-NMR Spektren:
Frequenz: 400 bzw. 100 MHz
Lösungsmittel: CDCl₃, 25°C
Varian Inova 400 Spektrometer
TMS-Standard

### Ergebnisse der Charakterisierung:

A) Schmelzpunkte ausgewählter erfindungsgemäßer Verbindungen (°C) (zur Benennung mit Trivialnamen und Struktur der Reste R₁-R₆: siehe Tabelle 1):

| | |
|---|---|
| 1-Allyfestuclavin | 81-81 |
| 1-Allyllysergol | 124-125 |
| 1-Allyluol | 203-205 |
| Ergotamin (Referenz) | 175-177 |
| 1-Allylergotamin | 179-181 |
| Dihydroergotamin (Referenz) | 237-238 |
| 1-Allyldihydroergotamin | 165-167 |
| Lisurid (Referenz) | 173-175 |
| 1-Allyllisurid | 72-74 |
| Tergurid (Referenz) | 206 |
| 1-Allyltergurid | 66-67 |

B) MS-Daten für 1-Allyltergurid [m/z (relative Intensität)]:
381 (10),380 (39), 308 (6), 307 (15), 265 (6), 264 (24), 263 (36), 249 (8), 221 (5), 220 (5), 209 (7), 208 (21), 207 (100), 195 (12), 194 (18), 100 (4), 74 (3).
C) NMR-Daten für 1-Allyl-(5*R,*8*S,*10*R*)-tergurid:

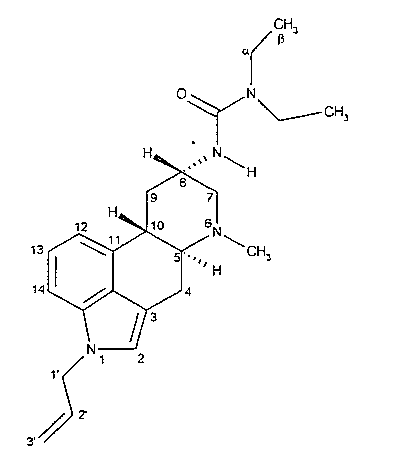

| | ¹³C NMR | | ¹H NMR | | | |
|---|---|---|---|---|---|---|
| Atom | δ | mult. | δ | ⁿH | mult.^{a} | J(Hz) |
| 2 | 121.73 | d | 6.774 | 1 | d | 1.7 |
| 3 | 110.84 | s | | | | |
| 4 | 26.91 | t | 2.667 | 1 | ddd | 19.6, 11.1, 1.7 |
| | | | 3.379 | 1 | dd | 14.6, 4.3 |
| 5 | 67.61 | d | 2.205 | 1 | ddd | 11.1, 9.7, 4.3 |
| 7 | 61.89 | t | 2.485 | 1 | dd | 11.7, 2.6 |
| | | | 2.874 | 1 | ddd | 11.7, 2.6., 2.4 |
| 8 | 44.95 | d | 4.282 | 1 | m | |
| 9 | 32.55 | t | 1.636 | 1 | ddd | 13.2, 13.0, 3.3 |
| | | | 2.796 | 1 | dddd | 13.2., 4.3, 2.6, 2.6 |
| 10 | 36.52 | d | 3.052 | 1 | m | |
| 11 | 133.52 | s | | | | |
| 12 | 112.83 | d | 6.888 | 1 | ddd | 6.9, 1.3, 0.9 |
| 13 | 122.73 | d | 7.158 | 1 | dd | 8.3, 6.9 |
| 14 | 107.12 | d | 7.100 | 1 | ddd | 8.3, 0.9, 0.8 |
| 15 | 133.68 | s | | | | |
| 16 | 126.71 | s | | | | |
| 14-CH₃ | 43.39 | q | 2.416 | 3 | s | |
| N-C=O | 156.57 | s | | | | |
| 1' | 48.91 | t | 4.674 | 2 | ddd | 5.5, 1.7, 1.5 |
| 2' | 133.87 | d | 5.990 | 1 | ddt | 17.0, 10.2, 5.5 |
| 3' | 117.00 | t | 5.124 | 1 | ddt | 17.0, 1.3, 1.7 |
| | | | 5.188 | 1 | ddt | 10.2, 1.3, 1.5 |
| α | 41.06 | t | 3.250 | 1 | dq | 19.5, 7.1 |
| | | | 3.347 | 1 | dq | 14.5, 7.1 |
| β | 13.85 | q | 1.152 | 3 | t | 7.1 |
| N-H | | | 5.572 | 1 | d | 8.2 |
| | | | | | | |

### Beispiel 2: Auf 5-HT_{2B}-Rezeptorgewebe basierender Funktionstest

Es handelte sich hierbei um einen in vitro Funktionstest zur Charakterisierung von 5-HT_{2B} Rezeptoren in einer Schweinelungenarterie. Die Präparation der Schweinelungenarterie wurde wie folgt durchgeführt: Kleine Zweige der Lungenarterie wurden seziert und vorsichtig von Organgewebe und Bindegewebe befreit. Bis zu sechs Ringe der Arterie (2-3 mm lang und 1,5-2 mm breit) wurden horizontal zwischen zwei L-förmigen Platinhaken aufgehängt (Durchmesser 150 µm) und in einem Organbad fixiert, das 10 ml modifizierte Krebs-Henseleit-Lösung der folgenden Zusammensetzung enthielt: 118 mM NaCl, 4,7 mM KCl, 2,5 mM CaCl₂, 1, 2 mM MgSO₄, 1,2 mM KH₂PO₄, 25 mM NaHCO₃ und 11 mM D-Glucose. Die Lösung wurde kontinuierlich mit einer Gasmischung aus 95% O₂ und 5% CO₂ durchströmt und bei einer konstanten Temperatur von 35°C gehalten. Die Präparationen wurden mit einem isometrischen Kraftumwandler verbunden (Hugo Sachs Elektronik, March, Deutschland), und Wechsel in der Spannung wurden kontinuierlich aufgezeichnet. Die bleibende Spannung im stationären Zustand wurde zu Beginn jedes Experiments jeweils auf 20 mN justiert. Während einer Anfangsstabilisierungszeit von 60 min wurde das Bad-Medium einmal alle 20 min gewechselt und die Spannung wiederholt auf 20 mN einjustiert. Die Arterienpräparationen wurden in Intervallen von 45 min einmal mit KC1 (30 mM) und dreimal mit Prostaglandin-F_{2α} (PGF_{2α}, 3µM) stimuliert, bis die Antwort durch Kontraktion konstant war. Die Unversehrtheit der Gefäßinnenhaut (Endothelium) wurde durch Messung des Umfangs der Endothelium-abhängigen Relaxation, die auf eine Bradykinin-Applikation (10 nM) folgt, funktional beurteilt. Nachdem sich die dritte PGF_{2α}-induzierte Kontraktion stabilisiert hatte, wurde die relaxierende Antwort auf 5-HT untersucht, indem eine kumulative Konzentrations-Antwort-Kurve in Abwesenheit und Gegenwart des Antagonisten ermittelt wurde. Abbildung 1 zeigt die Effekte auf die Relaxationsantwort bei Zugabe der erfindungsgemäßen Verbindungen 1-Allylergotamin und 1-Allyldihydroergotamin.
Zum Test auf agonistische Aktivität wurde die relaxierende Antwort auf die Testverbindung nach demselben Verfahren wie mit 5-HT untersucht. Die Konzentration des Agonisten wurde schrittweise jeweils an dem Zeitpunkt erhöht, an dem das Antwortsignal ein Plateau erreicht hatte. Das Plateau wurde üblicherweise innerhalb von 2-4 Minuten erreicht. Die Relaxationseffekte wurden als Prozentwert der PGF_{2α}-induzierten Kontraktion ausgedrückt. Die Antagonisten wurden 30 Minuten vor Beginn der Aufzeichnung der Agonistenkonzentrations-Antwort-Kurven zugegeben. Die Wirkungen der Antagonisten wurden in Ringsegmenten untersucht, die benachbart zu den Vergleichssegmenten waren.

Die antagonistische Wirkung der erfindungsgemäßen Verbindungen auf 5-HT_{2B}-Rezeptoren wurde durch diese Methode nachgewiesen(siehe Tabelle 1). Die selektive Wirkung auf 5-HT_{2A}-Rezeptoren bzw. 5-HT_{2B}-Rezeptoren wurde durch ein Vergleichsscreening bestätigt.

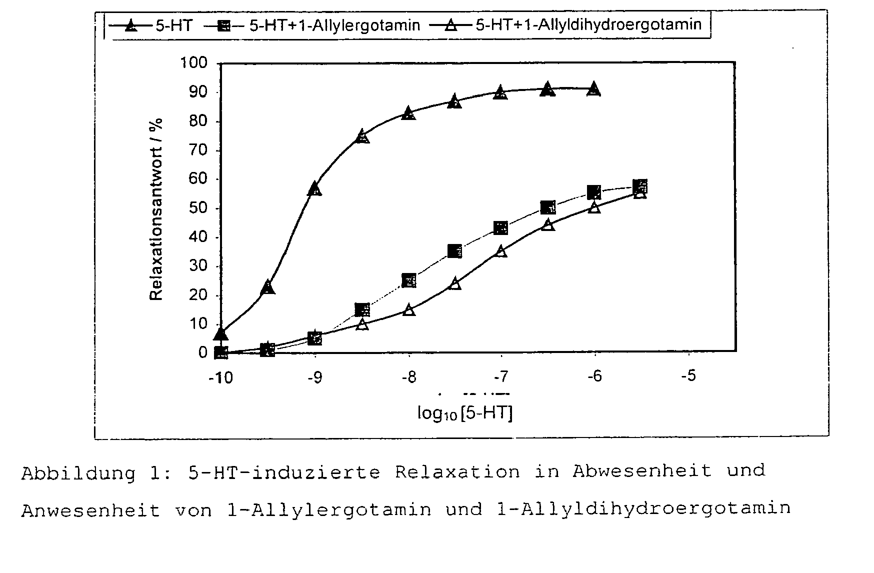

### Beispiel 3: Auf 5-HT_{2A}-Rezeptorgewebe basierender Funktionstest

Das folgende Beispiel beschreibt einen in vitro Funktionstest zur Charakterisierung von 5-HT_{2A}-Rezeptoren in Rattenschwanzarterien. Die Präparation der Rattenschwanzarterie wurde nach dem von Schöning beschriebenen Verfahren durchgeführt (Schöning et al., 2001, Die komplexe Wechselwirkung von Ergovalin mit 5-HT_{2A}-, 5-HT_{1B/1D}- und alpha₁-Rezeptoren in isolierten Arterien von Ratten und Guinea-Schweinen, J. Anim. Sci., 79, 2202-2209).

Männliche Wistar-Ratten (250-300 g) wurden durch Ersticken getötet. Die vordere Schwanzarterie wurde schnell seziert und von anhängendem Bindegewebe gereinigt. Ein Edelstahldraht (Durchmesser 0,3 mm) wurde in die Arterie eingeführt, um das Endothelium abzustreifen. Bis zu 20 zylindrische Segmente von 3-4 mm Länge wurden horizontal zwischen zwei L-förmigen Platinhaken aufgehängt (Durchmesser 150 µm) und in einem Organbad fixiert, das 20 ml modifizierte Krebs-Henseleit-Lösung der folgenden Zusammensetzung enthielt: 118 mM NaCl, 4,7 mM KCl, 2,5 mM CaCl₂, 1,2 mM MgSO₄, 1,2 mM KH₂PO₄, 25 mM NaHCO₃ und 10 mM D-Glucose. Die Lösung wurde kontinuierlich mit einer Gasmischung aus 95% O₂ und 5% CO₂ durchströmt und bei einer konstanten Temperatur von 37°C gehalten. Die Präparationen wurden mit einem isometrischen Kraftumwandler verbunden (W. Fleck, Mainz, Deutschland), der an einen TSE 4711-Umwandlungskoppler und einen Siemens C1016 Kompensographen gekoppelt wurde, um die Spannungsmodulation kontinuierlich aufzuzeichnen. Die bleibende Spannung wurde zu Beginn jedes Experiments auf 5 mN justiert. Während einer Gleichgewichtsperiode von 120 min wurden die Präparationen nach 60 min einmalig mit 5-HT (1 µM) stimuliert.
In diesen Experimenten zur Untersuchung der partiellen Agonisten wurden drei kumulative Konzentrations-Antwort-Kurven (KAK) für jedes Arteriensegment ermittelt. Die erste KAK wurde für 5-HT erhalten, die zweite, 60 min später folgende, für den partiellen Agonisten. Zum Schluß folgte die dritte KAK 10-15 min nach der zweiten KAK, ohne Waschen mit 5-HT und unter Einsatz der höchsten Agonistenkonzentration (0,3-3 µM). In zusätzlichen Experimenten wurden zwei KAK in einem Intervall von 60 min wie oben beschrieben ermittelt. Die erste KAK wurde für 5-HT ermittelt. Die zweite KAK wurde ermittelt, um den partiellen Agonisten in Gegenwart von Ketanserin zu testen. Die Präparationen wurden 30 min in einer Ketanserin-Lösung (3 nM) inkubiert. Die axiale Rechtsverschiebung der Kurven, die in Gegenwart von Ketanserin ermittelt wurde, wurde zu der Verschiebung ins Verhältnis gesetzt, die für die jeweilige Kontrollpräparation in Abwesenheit von Ketanserin ermittelt wurde. In diesen Experimenten mit Verwendung von Ketanserin wurden bei jedem Arteriensegment in einem Intervall von 90 min zwei getrennte KAK auf 5-HT ermittelt. Im Fall der Antagonisten wurden die Präparationen 60 min vor Ermittlung der zweiten Kurve inkubiert. Prazosin (0,1 µM) und Kokain (6 µM) waren während der Experimente in der Lösung vorhanden, um die α₁-Adrenozeptoren und die neuronale Aufnahme von 5-HT zu blockieren.

Durch den Test mit der oben beschriebenen Methode wurde nachgewiesen, dass die erfindungsgemäßen Verbindungen partielle oder vollständige Antagonisten der 5-HT_{2A}-Rezeptoren sind (Tabelle 1).

**Tabelle 1: Test auf antagonistische Wirkung bei 5-HT_{2A} und 5-HT_{2B}-Rezeptoren**

| Verbindung | Struktur (nach Formel I und II) | | | | | | | 5-HT_{2A} | | 5-HT_{2B} | | Selektivität HT_{2A} : HT_{2B} |
|---|---|---|---|---|---|---|---|---|---|---|---|---|
| | R₁ | R₂ | R₃ | R₄ | R₅ | R₆ | Bindung C⁹-C¹⁰ | Konz. (nM) | pA₂- Wert | Konz. (nM) | pA₂- Wert | |
| Lisurid (Referenz) | -CH₃ | -NHCON(C₂H₅)₂ | - | H | - | - | ungesättigt | 10 | 8,60 | 1 | 10,32 | 1 : 52 |
| 1-Allyllisurid | -CH₃ | -NHCON(C₂H₅)₂ | - | H | - | - | ungesättigt | 30 | 8,92 | 1000 | 8,17 | 6 : 1 |
| Tergurid (Referenz) | -CH₃ | -NHCON(C₂H₅)₂ | H | H | - | - | gesättigt | 300 | 8,38 | 10 | 8,49 | 1 : 1 |
| 1-Allyltergurid | -CH₃ | -NHCON(C₂H₅)₂ | H | H | - | - | gesättigt | 30 | 8,70 | 1000 | 7,67 | 11 : 1 |
| Festuclavin* (Referenz) | -CH₃ | H | H | -CH₃ | - | - | gesättigt | - | 6,63 | - | - | - |
| 1-Allylfestuclavin | -CH₃ | H | H | -CH₃ | - | - | gesättigt | 10-30 | 7,89 | 1000 | 6,74 | 14 : 1 |
| Lysergol* (Referenz) | -CH₃ | H | - | -CH₂OH | - | - | ungesättigt | - | 6,88 | - | - | - |
| 1-Allyllysergol | -CH₃ | H | - | -CH₂OH | - | - | ungesättigt | 3-100 | 8,45 | 1000 | 7,61 | 7 : 1 |
| 1-Allyluol | -CH₃ | H | -OCH₃ | -CH₂OH | - | - | gesättigt | 100 | 7,36 | 1000 | 7,03 | 2 : 1 |
| 1-Allyldihydro-ergotamin | -CH₃ | H | - | Rest der Formel (II) | -CH₃ | Benzyl | gesättigt | 10 | 7,65 | 30 | 9,30 | 1 : 45 |
| 1-Allylergotamin | -CH₃ | H | - | Rest der Formel (II) | -CH₃ | Benzyl | ungesättigt | 10 | 7,85 | - | 9,11** | 1 : 18 |

| | | | | | | | | | | | | |
|---|---|---|---|---|---|---|---|---|---|---|---|---|
| * Messwerte aus H. Pertz (1996) Planta Med. 62, 387-392 ** -logKp, berechnet nach Kenakin | | | | | | | | | | | | |

## Patentansprüche

1. Allyl-Ergotalkaloid-Derivate der allgemeinen Formel (I) 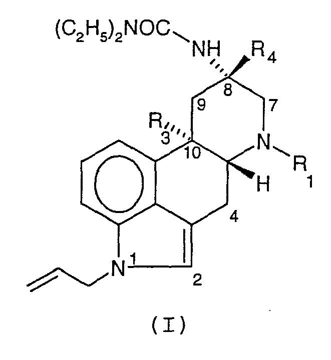 in welcher
R₁ für eine Ethyl-, n-Propyl-, i-Propyl- oder Allylgruppe,
R₃ für Wasserstoff oder eine -OCH₃-Gruppe, und
R₄ für Wasserstoff, eine Methyl-, Ethyl-, n-Propyl-, i-Propyl- oder -CH₂OH-Gruppe steht
und die Bindung zwischen den C-Atomen 9 und 10 entweder eine Einfachbindung oder eine Doppelbindung bei gleichzeitigem Wegfall des Restes R₃ ist.

2. Verfahren zur Herstellung der Verbindungen der allgemeinen Formel (I) gemäß Anspruch 1, **dadurch gekennzeichnet, dass** eine Verbindung der allgemeinen Formel (III) 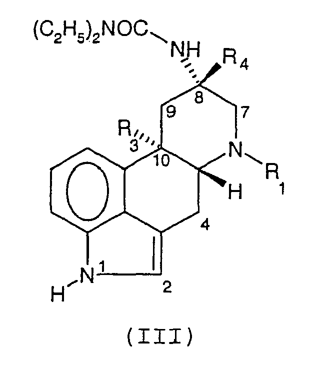 in welcher
R₁ für eine Ethyl-, n-Propyl-, i-Propyl- oder Allylgruppe,
R₃ für Wasserstoff oder eine -OCH₃-Gruppe, und
R₄ für Wasserstoff, eine Methyl-, Ethyl-, n-Propyl-, i-Propyl- oder -CH₂OH-Gruppe steht und
die Bindung zwischen den C-Atomen 9 und 10 entweder eine Einfachbindung oder eine Doppelbindung bei gleichzeitigem Wegfall des Restes R₃ ist,
mit Allylbromid in CH₂Cl₂ gegebenenfalls unter Zugabe von Tetraethylammoniumhydroxid und NaOH umgesetzt wird.

3. Pharmazeutische Zusammensetzung, enthaltend mindestens eine Verbindung der Formel (I) gemäß Anspruch 1 sowie einen oder mehrere pharmakologisch unbedenkliche Hilfsstoffe und/oder Trägerstoffe.

4. Verwendung der Verbindungen der Formel (I) gemäß Anspruch 1 und ihrer pharmazeutisch verträglichen Salze zur Herstellung eines Arzneimittels zur Prophylaxe und/oder Behandlung von Migräne.

5. Verwendung der Verbindungen der Formel (I) gemäß Anspruch 1 und ihrer pharmazeutisch verträglichen Salze zur Herstellung eines Arzneimittels zur Behandlung von M. Parkinson.

6. Verwendung der Verbindungen der Formel (I) gemäß Anspruch 1 und ihrer pharmazeutisch verträglichen Salze zur Herstellung eines Arzneimittels zur Prophylaxe und/oder Behandlung der Thrombozytenaggregation.

7. Verwendung der Verbindungen der Formel (I) gemäß Anspruch 1 und ihrer pharmazeutisch verträglichen Salze zur Herstellung eines Arzneimittels zur Prophylaxe und/oder Behandlung von psychischen Erkrankungen.

8. Verwendung der Verbindungen der Formel (I) gemäß Anspruch 1 und ihrer pharmazeutisch verträglichen Salze zur Herstellung eines Arzneimittels zur Behandlung von Erkrankungen des Nervensystems.

## Claims

1. Allyl ergot alkaloid derivatives of the general formula (I) 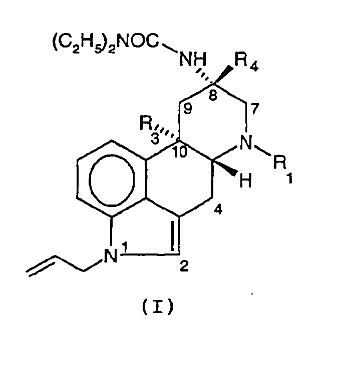 wherein
R₁ represents an ethyl, n-propyl, i-propyl, or allyl group,
R₃ is hydrogen or an -OCH₃ group, and
R₄ represents hydrogen, a methyl, ethyl, n-propyl, i-propyl, or -CH₂OH group,
and the bond between C atoms 9 and 10 is either a single or a double bond while the residue R₃ is omitted.

2. A method for producing the compounds of the general formula (I) according to claim 1 wherein a compound of the general formula (III) 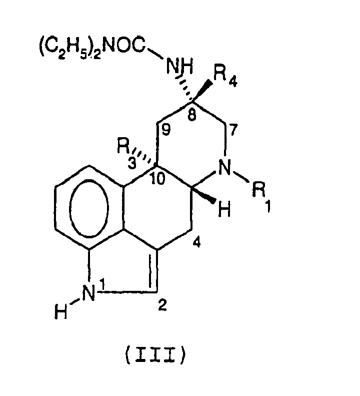 wherein
R₁ represents an ethyl, n-propyl, i-propyl, or allyl group,
R₃ is hydrogen or an -OCH₃ group, and
R₄ represents hydrogen, a methyl, ethyl, n-propyl, i-propyl, or -CH₂OH group, and
the bond between C atoms 9 and 10 is either a single or a double bond while the residue R₃ is omitted, is reacted with allyl bromide in CH₂Cl₂, optionally adding tetraethyl ammonium hydroxide and NaOH.

3. A pharmaceutical composition containing at least one compound of the general formula (I) according to claim 1 as well as one or several pharmacologically safe adjuvants and/or substrates.

4. Use of the compounds of the general formula (I) according to claim 1 and their pharmaceutically tolerable salts for producing a pharmaceutical for the prevention and/or treatment of migraine.

5. Use of the compounds of the general formula (I) according to claim 1 and their pharmaceutically tolerable salts for producing a pharmaceutical for the treatment of Parkinson's disease.

6. Use of the compounds of the general formula (I) according to claim 1 and their pharmaceutically tolerable salts for producing a pharmaceutical for the prevention and/or treatment of thrombocyte aggregation.

7. Use of the compounds of the general formula (I) according to claim 1 and their pharmaceutically tolerable salts for producing a pharmaceutical for the prevention and/or treatment of psychic diseases.

8. Use of the compounds of the general formula (I) according to claim 1 and their pharmaceutically tolerable salts for producing a pharmaceutical for the treatment of diseases of the nervous system.

## Revendications

1. Dérivés d'allyl-alcaloïdes ergot de formule générale (I) 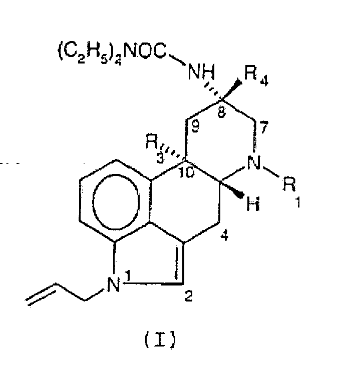 dans laquelle
R₁ représente un groupe éthyle, n-propyle, i-propyle ou allyle,
R₃ représente de l'hydrogène ou un groupe OCH₃, et
R₄ représente de l'hydrogène, un groupe méthyle, éthyle, n-propyle, i-propyle ou CH₂OH
et la liaison entre les atomes C 9 et 10 est soit une liaison simple ou une double liaison en cas de suppression simultanée du radical R₃.

2. Procédé pour la fabrication des composés de formule générale (I) selon la revendication 1, **caractérisé en ce qu'**un composé de formule générale (III) 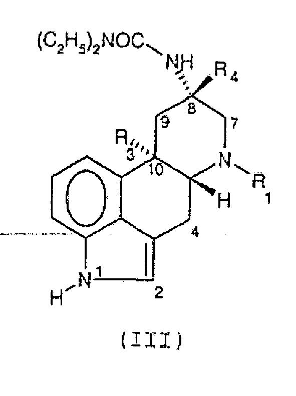 dans laquelle
R₁ représente un groupe éthyle, n-propyle, i-propyle ou allyle,
R₃ représente de l'hydrogène ou un groupe OCH₃, et
R₄ représente de l'hydrogène, un groupe méthyle, éthyle, n-propyle, i-propyle ou CH₂OH
et
dans laquelle la liaison entre les atomes C 9 et 10 est soit une liaison simple ou une double liaison en cas de suppression simultanée du radical R₃,
est transformé avec du bromure d'allyle en CH₂Cl₂ en ajoutant, le cas échéant, de l'hydroxyde de tétraéthylammonium et du NaOH.

3. Composition pharmaceutique, contenant au moins un composé de la formule (I) selon la revendication 1, ainsi qu'un ou plusieurs adjuvants et/ou substances de support pharmacologiquement neutre.

4. Utilisation des composés de la formule (I) selon la revendication 1 et de leurs sels pharmaceutiquement compatibles pour fabriquer un médicament pour la prophylaxie et/ou le traitement de la migraine.

5. Utilisation des composés de la formule (I) selon la revendication 1 et de leurs sels pharmaceutiquement compatibles pour fabriquer un médicament pour le traitement de la maladie de Parkinson.

6. Utilisation des composés de la formule (I) selon la revendication 1 et de leurs sels pharmaceutiquement compatibles pour fabriquer un médicament pour la prophylaxie et/ou le traitement de l'agrégation des thrombocytes.

7. Utilisation des composés de la formule (I) selon la revendication 1 et de leurs sels pharmaceutiquement compatibles pour fabriquer un médicament pour la prophylaxie et/ou le traitement de maladies psychiques.

8. Utilisation des composés de la formule (I) selon la revendication 1 et de leurs sels pharmaceutiquement compatibles pour fabriquer un médicament pour le traitement de maladies du système nerveux.
